# EUROPEAN PATENT SPECIFICATION

(11) **EP 2 424 573 B1**
(45) Date of publication and mention of the grant of the patent: **16.01.2019**
(21) Application number: 10718133.1
(22) Date of filing: 27.04.2010
(51) Int. Cl.: A61K 49/00, A61K 51/04, C07B 59/00, C07C 323/58

(54) **LABELED MOLECULAR IMAGING AGENTS, METHODS OF MAKING AND METHODS OF USE**
MARKIERTE MOLEKULARE BILDGEBUNGSMITTEL, HERSTELLUNGS- UND ANWENDUNGSVERFAHREN
AGENTS D'IMAGERIE MOLÉCULAIRE MARQUÉS, LEURS PROCÉDÉS DE FABRICATION ET MÉTHODES D'UTILISATION

(30) Priority: 27.04.2009 US 430573
(43) Date of publication of application: 07.03.2012
(73) Proprietor: General Electric Company, Schenectady, NY 12345 (US)
(72) Inventor: SYUD, Faisal, Ahmed, Niskayuna, New York 12309 (US); LEE, Brian, Duh-Lan, Niskayuna, New York 12309 (US); SCHAFFER, Paul, Niskayuna, New York 12309 (US); ZHANG, Rong, Niskayuna, New York 12309 (US); WEBSTER, Jack, Mathew, Niskayuna, New York 12309 (US); HUNTINGTON, Jennifer, Niskayuna, New York 12309 (US); AMARASINGHE, Kande, Kankanamalage, Dayara, Niskayuna, New York 12309 (US)
(74) Representative: Serjeants LLP
(86) International application number: PCT/EP2010/055629
(87) International publication number: WO 2010/125068

(56) References cited:
- WO-A2-2006/030291
- CHENG Q ET AL: "Monolayer and epi-fluorescence microscopy studies of amino acid derivatized diacetylene lipids", THIN SOLID FILMS, ELSEVIER-SEQUOIA S.A. LAUSANNE, CH, vol. 345, no. 2, 21 May 1999 (1999-05-21), pages 292-299, XP004177543, ISSN: 0040-6090, DOI: DOI:10.1016/S0040-6090(98)01608-3
- LIU, Y. ET AL: "Synthesis of L-cystine modified cyclodextrin monomers and dimers with primary-side versus secondary-side and their molecular binding behaviors", 2008, SUPRAMOLECULAR CHEMISTRY , 20(7), 609-617 CODEN: SCHEER; ISSN: 1061-0278, XP8129496, the whole document
- MAYA DAMIANOVICH ET AL: "ApoSense: a novel technology for functional molecular imaging of cell death in models of acute renal tubular necrosis", EUROPEAN JOURNAL OF NUCLEAR MEDICINE AND MOLECULAR IMAGING, SPRINGER, BERLIN, DE, vol. 33, no. 3, 1 March 2006 (2006-03-01), pages 281-291, XP019333018, ISSN: 1619-7089, DOI: DOI:10.1007/S00259-005-1905-X
- RESHEF AYELET ET AL: "Targeting cell death in vivo in experimental traumatic brain injury by a novel molecular probe.", JOURNAL OF NEUROTRAUMA JUN 2008 LNKD- PUBMED:18447626, vol. 25, no. 6, June 2008 (2008-06), pages 569-580, XP002611216, ISSN: 0897-7151
- DUN Y ET AL: "Expression of the cystine-glutamate exchanger (xc -) in retinal ganglion cells and regulation by nitric oxide and oxidative stress", CELL AND TISSUE RESEARCH, SPRINGER, BERLIN, DE, vol. 324, no. 2, 1 May 2006 (2006-05-01), pages 189-202, XP019346702, ISSN: 1432-0878, DOI: DOI:10.1007/S00441-005-0116-X

## Description

### BACKGROUND

The invention relates generally to labeled molecular imaging agents and more particularly to imaging agents that are taken up by the cells via the cystine/glutamate transporter.

The cystine/glutamate transporter is not typically expressed or has extremely low expression in most tissues, but is upregulated in cells exposed to oxidative stress. Cystine, which comprises two disulfide-linked cysteine amino acids, is a natural substrate for this transporter. The cystine/glutamate antiporter (xc- system) is an amino acid transporter (designated SLC7A11) made up of two protein subunits; 4F2hc/CD98, a common subunit for a few classes of transport systems, and xCT, which is specific to the cystine/glutamate exchanger. The effect of upregulation of the transporter is an increase in cystine uptake; which is then reduced to cysteine inside the cell. Intracellular cysteine is the rate limiting substrate for glutathione synthesis. Glutathione is the cells primary anti-oxidant to defend against oxidative stress. Intracellular cysteine is incorporated into one of two pathways, glutathione synthesis or protein synthesis.

Fluorescently-labeled cystine compounds comprising fluorescent labels attached to both of the amine groups of cystine are known in the art. One example is N,N'-Didansyl-L-cystine (DDC), commercially available from Sigma (catalogue #D0625), which has the following structure:

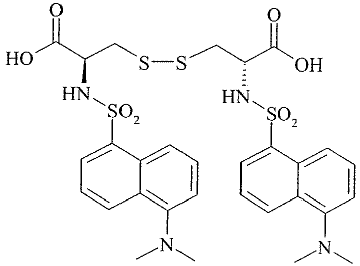

Another examples is DiBodipy L-cystine (DBC) is a commercially available product from Invitrogen (catalog #B20340), which is sold for the purpose of reversible thiol labeling of nucleotides, proteins and cells via a disulfide exchange reaction at acidic conditions. DBC has the structure shown below.

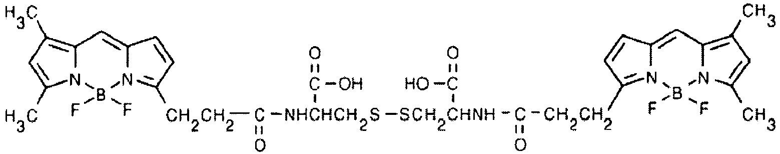

Radiolabelled versions of cystine are also known in the art. For example, cystine labeled with ^{99m}Tc is known. Tubis and Endow (1968 Int J Appl Rad Isotop; 19: 835-840) discloses the direct reaction of cysteine with ^{99m}TcO₄⁻ wherein the -SH group of cysteine reduces the ^{99m}TcO₄⁻ while being simultaneously oxidized to cystine. Also, cystine labeled with ³⁵S is known and has the following structure: 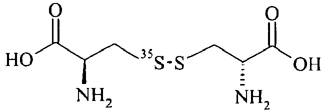 ³⁵S has been used for example in animal radiotracer studies (see e.g. Hwang et al 1980 J Neurochem; 35: 417-424).

The concept of molecular imaging promises specific contrast enhancement of molecular signatures of pathology and requires targetable biomarkers that are specifically regulated in certain pathological indications. While such a specific molecular contrast agent could have great utility for imaging and diagnosing disease; validation of a truly specific biomarker has proven to be very difficult. Even if an agent to such a specific biomarker is created, the market for such an agent will be limited to the prevalence of this indication. Therefore there is great interest in developing molecular contrast agents that can be utilized to image a variety of pathological indications. Most imaging agents target specific tissue or cell types, or specific therapies, or they degrade rapidly over time. One example of an agent that is directed at broader applications is 18-F-fluorodeoxyglucose (FDG) that makes use of the glucose transporter. ¹⁸F-FDG is preferentially taken up by cells that have an increased requirement for glucose, and then is trapped inside the cell. FDG can be used clinically for the diagnosis, staging and monitoring of many cancers as well as monitoring metabolism in the heart and brain. ¹⁸F-FDG is not a substrate for sodium-dependent glucose transporters found in the kidney tubules, which prevents its renal resorption and enhances clearance

In vivo oxidative stress is recognized as an indicator of cellular stress. Efforts to image this stress have involved imaging animals using electron paramagnetic resonance (EPR). EPR is a technique for detecting unpaired electrons as would occur with the creation of free radicals in oxidative stress. Essentially an agent is used which is considered to be an EPR probe which is sensitive to organ antioxidative activity as a measure of oxidative stress.

Others have also looked at using a 13-C-glycine chemical shift MRI to detect glycine uptake and conversion to glutathione in an animal model of chemotherapy treatment of tumors in vivo. Still others, have developed imaging agents to detect apoptotic cells in vivo for monitoring chemotherapy treatment e.g. labelled Annexin V which is a rather large protein, Aposense by Neurosurvival Technologies which is a family of small molecules which is reported to enter specifically into only apoptotic cells (see for instance Damianovich et al., Eur J Nucl Med Mol Imaging (2006) 33:281-291).

### SUMMARY OF THE INVENTION

The imaging agents and methods of the invention take advantage of the cellular amino acid transporter (cystine/glutamate antiporter, xc-), which is activated under conditions of cellular oxidative stress. These labeled molecular imaging agents and methods of the invention provide several benefits, including their use in a wide variety of diagnostic and therapeutic monitoring applications; they are small molecules and comprise labeled variants of a natural compound found in the body, and would be administered at tracer levels with physiological concentrations well below those that generate toxic response, therefore toxicity/immune response is expected to be low; and because the imaging agents act as a transporter substrate, the agents benefit from amplification of the signal because the molecular imaging agent is trapped once inside the cell, unlike other molecular binders in which the imaging agent's signal is limited to the stoichiometric binding of a cell surface epitope. The labeled substrates of the invention for the cystine/glutamate transporter may also be used to introduce compounds in a target for therapeutic purposes.

As a labeled substrate of the cystine/glutamate transporter, these imaging agents may be used for any disease or condition that relates to increased oxidative stress. For example, the imaging agents may be used image apoptotic cells in vivo. Such noninvasive monitoring of apoptotic cell death is useful, for example without limitation, to monitor chemotherapy effectiveness, tissue damage due to ischemia/stroke, traumatic injury, and transplant rejection. Imaging of oxidative stress is also useful for diagnosing and monitoring inflammatory diseases or any pathological indication that includes oxidative stress or tissue damage.

Additionally, upregulation of the cystine/glutamate transporter is also associated with chemotherapy resistance in some tumors. Therefore, non-invasive imaging of tumors with basal high cystine uptake could result in identification of tumors likely to be resistant to certain therapies; which could result in efficacious changes in treatment regimens.

An example of the method of the invention, for detecting oxidative stress in cells generally comprises: introducing an imaging agent comprising a labeled cystine into a cystine/glutamate antiporter of the cells; allowing the intracellular labeled cystine to be reduced into a labeled cysteine; detecting the labeled cysteine in the cell. For example, for some applications, the labeled cystine may be detected in apoptotic cells. In some examples of the method, the labeled cystine used in the method for detecting oxidative stress is selected from one of structures I, II, II and IV as defined more specifically hereunder.

Another embodiment of the imaging agent of the invention generally comprises, a labeled small molecule substrate of a cystine/glutamate transporter (xc-) according to the claims, such as Structures I, II, III, IV, V and VI, defined more specifically hereunder.

An example of a method for imaging of the invention generally comprises: using a cystine compound such as, structures I, II, III and IV as defined more specifically hereunder, wherein the compound is detected using, fluorescence microscopy, laser-confocal microscopy, cross-polarization microscopy, optical imaging, nuclear scintigraphy, positron emission tomography, single photon emission computed tomography.

### BRIEF DESCRIPTION OF THE FIGURES

These and other features, aspects, and advantages of the present invention will become better understood when the following detailed description is read with reference to the accompanying drawings in which like characters represent like parts throughout the drawings, wherein:

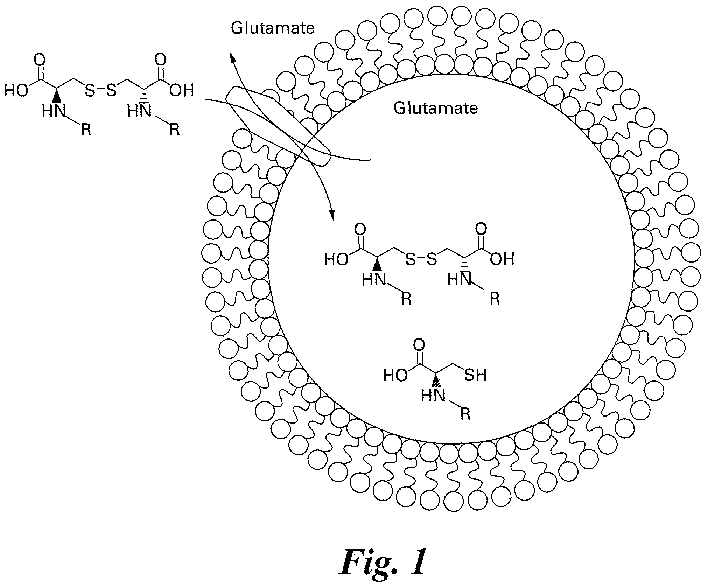

FIG 1 is a flow diagram of an embodiment of the imaging agent of the invention being transported into a cell via the cystine/glutamate antiporter.

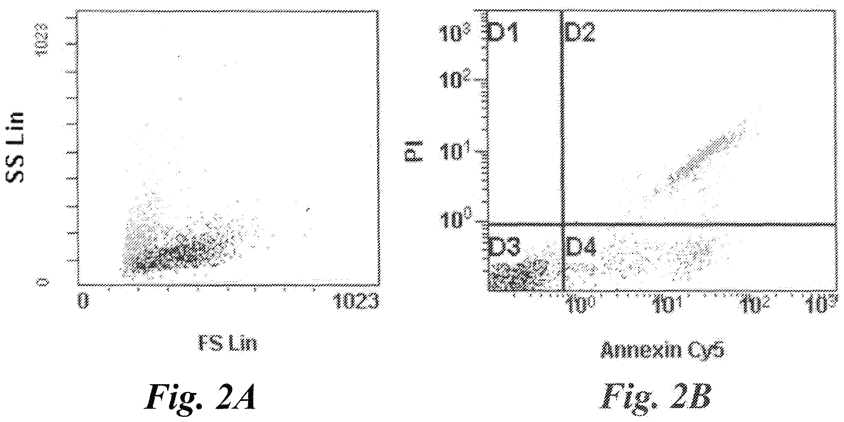

FIG 2A is a forward scatter/side scatter plot showing some late apoptotic Jurkat cells (light gray) with a different granularity than non-apoptotic Jurkat cells (darker gray). FIG 2B is a multi-quadrant scatter plot showing AnnexinV and propdium iodide (PI) negative cells in quadrant D3 and represent non-apoptotic cells; showing Annexin V-Cy5 positive and propidium iodide (PI) negative cells in Quandrant D4 representing early apoptotic cells; and showing Annexin V positive, cells in Quandrant D2 representing late apoptotic and necrotic cells

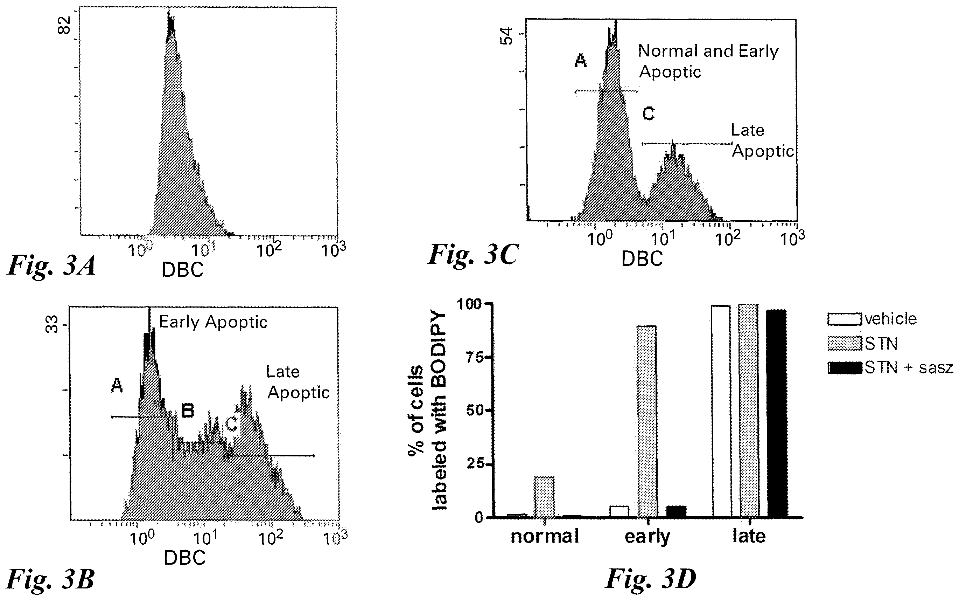

FIG 3A-D shows results from flow cytometric analysis of DBC uptake in Jurkat cells with and without staurosporine (STN) to induce oxidative stress/apoptosis and with and without sulfasalazine (sasz) to inhibit the cystine/glutamate transporter. FIG 3A is a graph showing untreated cells demonstrating some uptake or nonspecific binding of the DBC molecule. FIG 3B is a graph showing that staurosporine induced cells have three subpopulations of cells, low intensity DBC fluorescence representing normal cells, intermediate intensity representing early apoptotic cells and high intensity representing late apoptotic cells. FIG 3C is a graph showing that staurosporine induced cells that are exposed to the cystine/glutamate transporter inhibitor sulfasalazine (sasz) have a different result; a population of cells with intermediate DBC staining represents late apoptotic cells, while Early apoptotic cells are in the low DBC intensity population. FIG 4D is a bar chart showing the percentage of cells labeled greater than baseline DBC staining for normal, early apoptotic and late apoptotic cells (as determined by AnnexinV and PI staining).

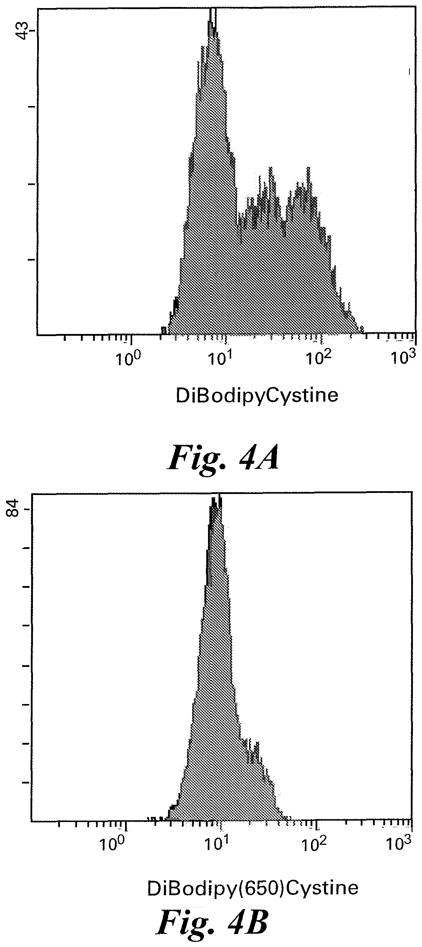

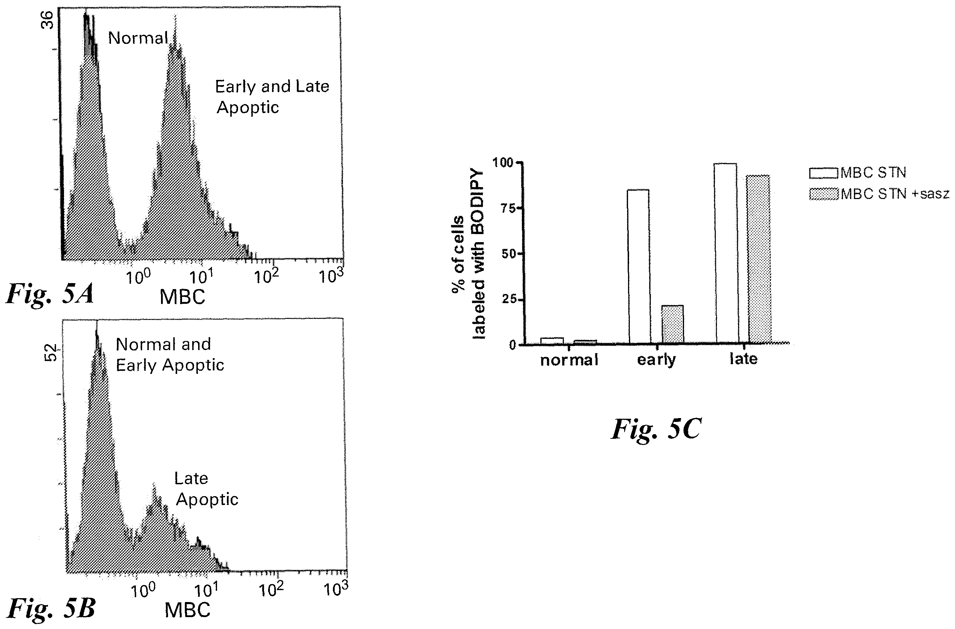

FIGs. 4A shows uptake of DBC in Jurkat cells treated with STN for 18 hours. FIG 4B shows a comparison of the same cells incubated with DiBodipy(650) Cystine (DBC(650)). FIG 5A-C shows results from flow cytometric analysis of MonoBodipyCystine (MBC) uptake in Jurkat cells with Staurosporine (STN) to induce oxidative stress/apoptosis with and without sulfasalazine (sasz) to inhibit the cystine/glutamate transporter. FIG 5A is a graph showing that staurosporine induced cells have two subpopulations of cells, no MBC fluorescence representing normal cells and high MBC intensity representing early and late apoptotic cells. FIG 5B is a graph showing that staurosporine induced that are exposed to the cystine/glutamate transporter inhibitor sulfasalazine (sasz) have a different result; a population of cells with intermediate MBC staining represents late apoptotic cells, while early apoptotic cells show no MBC fluorescence intensity. FIG 5C is a bar chart showing the percentage of cells labeled greater than baseline MBC staining for normal, early apoptotic and late apoptotic cells (as determined by AnnexinV and PI staining).

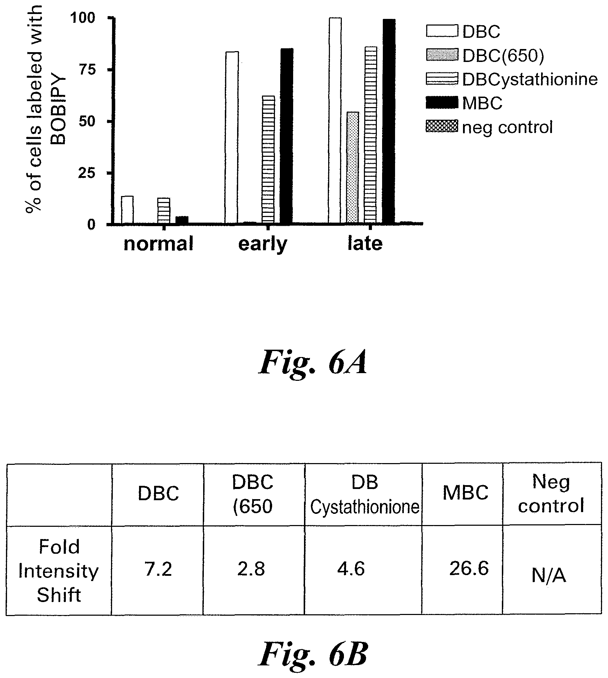

FIG 6A is a bar chart showing the percentage of normal, early apoptotic and late apoptotic cells (as determined by AnnexinV and PI staining) cells labeled with five different fluorescent agents: DBC, DBC(650), DiBodipyCystathionine (DBCystathionine), MBC and a negative control fluorescent molecule (Bodipy-FL C5). FIG 6B is a table showing the mean fold intensity shift for early apoptotic cells that are labeled with each of the five agents shown in FIG 6A.

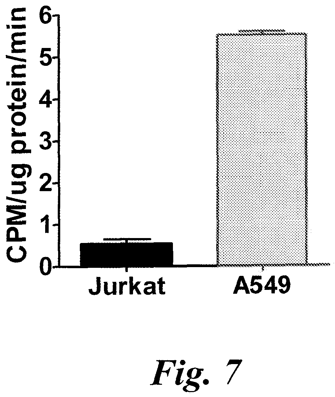

FIG 7 is a bar graph showing the uptake of monoAO-[¹⁸F]-FBA-Cystine in Jurkat and A549 cells in culture.

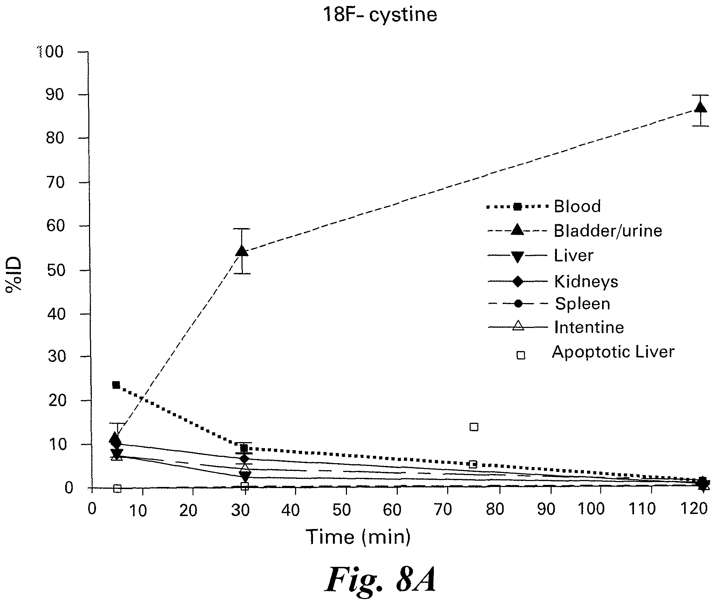

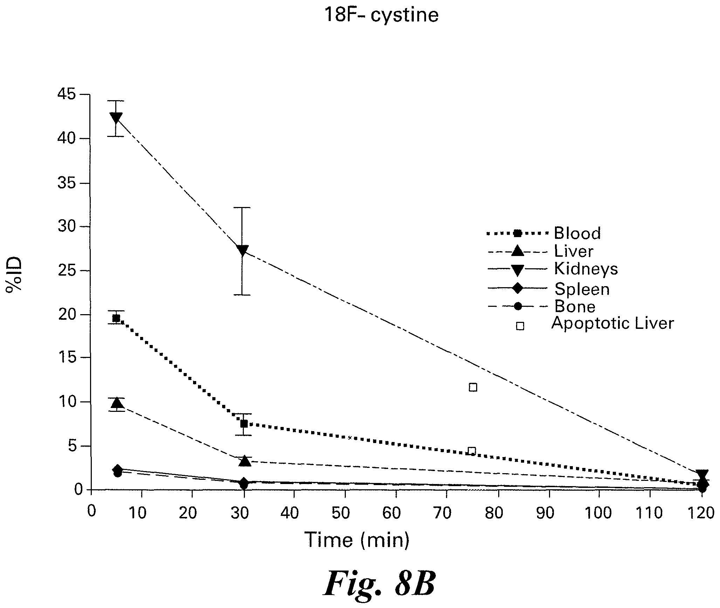

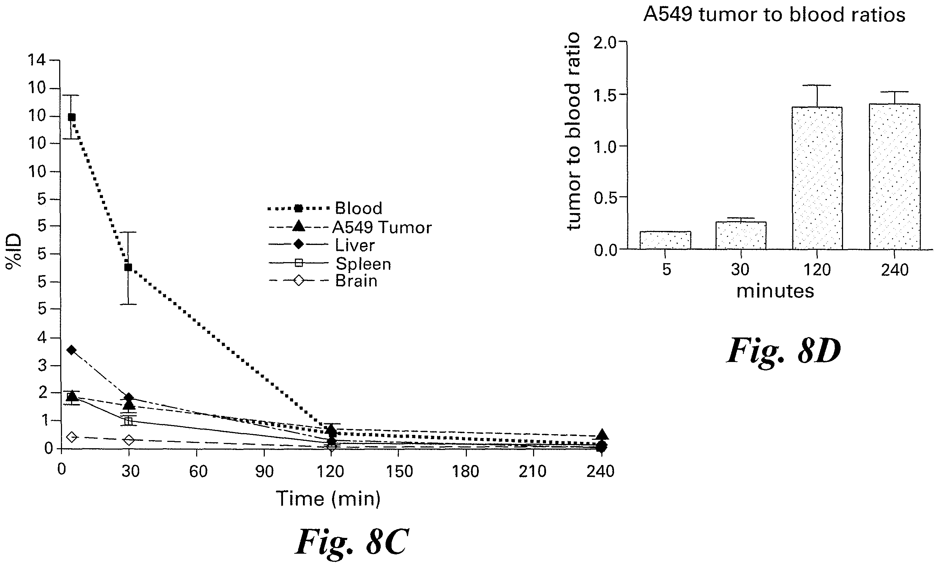

FIG 8A is a graph showing the biodistribution results in naive Balb-c mice in %ID/organ. FIG 8B is a graph showing the %ID/gram of the same data shown in FIG 8A. FIG 8C is a graph showing biodistribution results in nude mice with A549 xenograft tumors in % ID/gram. Figure 8D shows tumor to blood ratios at each timepoint and is derived from the same data shown in 8C.

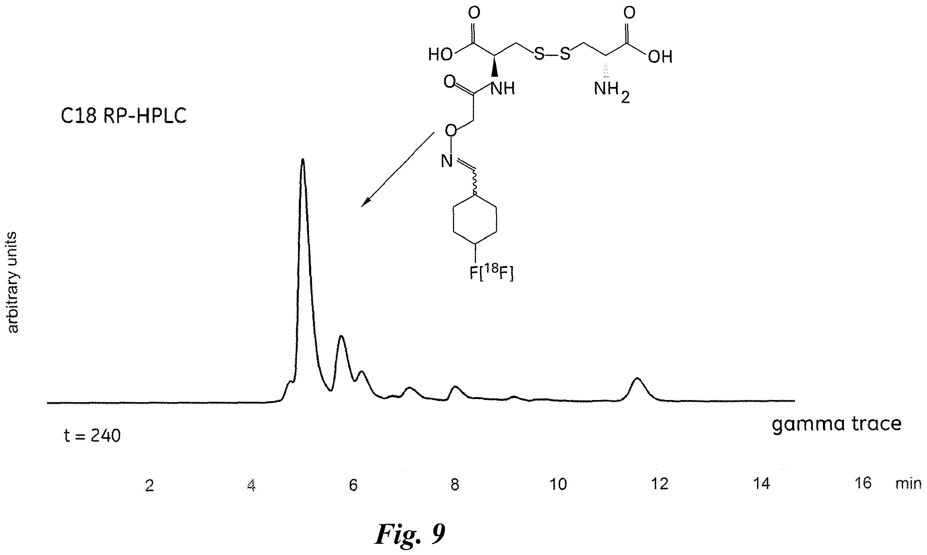

FIG 9 is a graph showing the stability of the monoAO-[¹⁸F]-FBA-Cystine molecule over time.

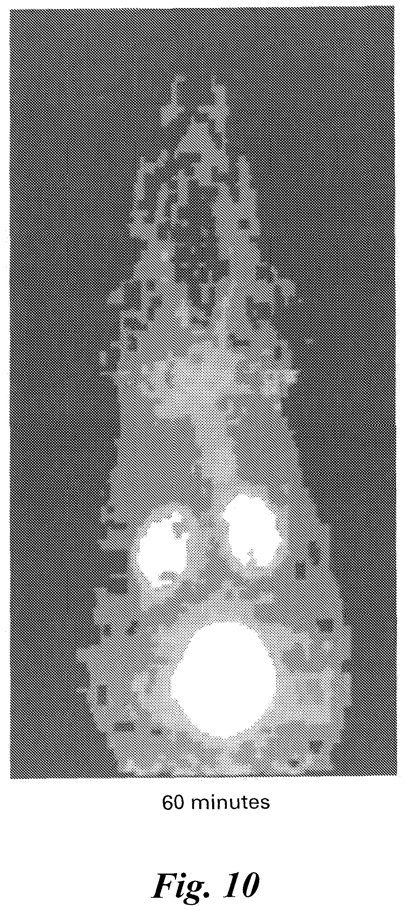

FIG 10 is an image showing the ¹⁸F-AO-FB-Cystine in a PET image in a naive mouse at 60 minutes post injection.

### DETAILED DESCRIPTION OF THE INVENTION

In one aspect, the present invention provides an imaging agent comprising a labeled cystine compound having structure I, 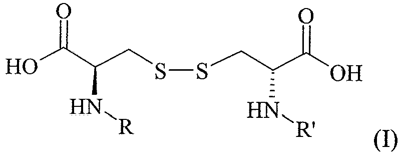 wherein one of R and R' comprises a label selected from a Bodipy fluorescent dye or a radioisotopic label, and the other of R and R' is hydrogen.

As used herein the term "imaging agent" is intended to encompass compounds that may be detected using either in vitro or in vivo imaging techniques.

As used herein, the term "fluorescent label" includes, fluorescent imaging agents and fluorophores, that are chemical compounds, which when excited by exposure to a particular wavelength of light, emit light at a different wavelength. Fluorophores may be described in terms of their emission profile, or color, and are the component of a molecule that causes the molecule to be fluorescent. It is typically a functional group that absorbs energy of a specific wavelength or range of wavelengths and re-emit energy at different but equally specific wavelengths or ranges.

As used herein, the term "radioisotopic label" includes, radioisotopes that are used in a compound to trace or visualize the compound, or the mechanism of a chemical reaction, in a chemical or biological process, or biological substances, organisms and systems. Such labels are useful, for example, in connection with imaging and detection systems.

Generally, the imaging agents of the invention comprise labeled cystine according to the claims. As shown in FIG 1, the imaging agents serve as a substrate for the cystine/glutamate transporter. Cystine labeled at a free amine is a substrate for the cystine/glutamate transporter. After transport into the cell, R-cystine is reduced to an R-cysteine and an unlabeled cysteine. R-cysteine is not metabolized and cannot exit via the transporter. Therefore it is retained in a cell that is responding to oxidative stress.

As used herein, the term "cystine/glutamate transporter" is used interchangeably with, and includes, the terms cystine/glutamate antiporter, cystine/glutamate exchanger, cystine transporter, xc(-), Xc(-), system xc(-), and amino acid transport system Xc(-). The transport system comprises dimer of two proteins and includes, but is not limited to: protein xCT and protein CD98 (4F2hc, heavy chain of the 4F2 surface antigen, SLC3A2); protein xCT which is the subunit specific to the xc(-) system; protein CD98 which is a subunit common to a number of transporters with different substrates; and protein xCT that may also dimerize with rBAT, another subunit common to multiple transporters.

The imaging agent of the invention may be detected by its emitted signal. The method of detection of the compounds may include, fluorescence microscopy, laser-confocal microscopy, cross-polarization microscopy, optical imaging, nuclear scintigraphy, positron emission tomography, and single photon emission computed tomography.

Where the imaging agent of the invention comprises a labeled cystine compound comprising a fluorescent dye according to the claims, suitable methods of detection include fluorescence microscopy, laser-confocal microscopy, cross-polarization microscopy, and optical imaging. The imaging agents of the invention comprises labeled cystine compounds according to the claims that comprise fluorescent labels where labeling occurs at the amine groups of the cystine. examples include: green fluorescent cystine molecules such as MonoBodipy(FL)-Cystine; red fluorescent cystine molecules such as MonoBodipy(650)-Cystine. Fluorescent labels, such as Bodipy fluorescent dyes, may be particularly suited for optical in vivo imaging and in vitro detection of cellular oxidative stress.

A particular labeled cystine compound comprising a fluorescent label has structure IV: 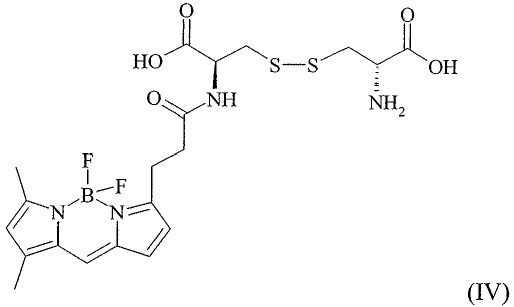 This compound is also referred to herein as MonoBodipyCystine (MBC).

Conjugation of a fluorescent label to cystine can be carried out by synthetic chemistry techniques well known to the skilled person. For example, imaging agents labeled comprising a fluorescent label suitable for optical imaging and their preparation are reviewed by Licha in "Contrast Agents for Optical Imaging" in Optical, Ultrasound, X-Ray and Radiopharmaceutical Imaing, Springer 2002, Krause Ed.

Where the imaging agent of the invention comprises a labeled cystine compound comprising a radioisotopic label, suitable methods of detection include nuclear scintigraphy, positron emission tomography and single photon emission tomography. Preferred radioisotopic labels for positron emission tomography include, ¹¹C, ¹³N, ¹⁵O, ¹⁷F, ¹⁸F, ⁷⁵Br, ⁷⁶Br or ¹²⁴I. Most preferred positron-emitting radioactive non-metals are ¹¹C, ¹³N, ¹⁸F and ¹²⁴I, especially ¹¹C and ¹⁸F, most especially ¹⁸F. A preferred radioisotopic labels for single photon emission tomography is a gamma-emitting radioactive halogen. Preferred gamma-emitting radioactive halogens are ¹²³I, ¹³¹I and ⁷⁷Br, especially ¹²³I. One or more of the imaging agents of the invention comprises prothstetic groups to enable radioisotope labeling where labeling occurs at the amine groups of the cystine. examples include: aminoxy(AO) derivatives of cystine that are labeled with ¹⁸F-fluorobenzaldehyde.

The labeled substrates of the invention for the cystine/glutamate transporter may also be used to introduce labeled compounds, such as, a cystine substrate labeled with ¹³¹I, into a target for therapeutic purposes.

In a preferred aspect, the imaging agent of the invention comprises a labeled cystine compound having structure II: 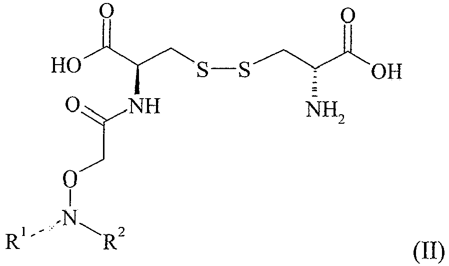 wherein.
R¹ comprises a radioisotopic label, R² is hydrogen when the dotted bond is a single bond; and,
R² is absent when the dotted bond is a double bond.

A particular labeled cystine compound of structure II has the more specific structure III:

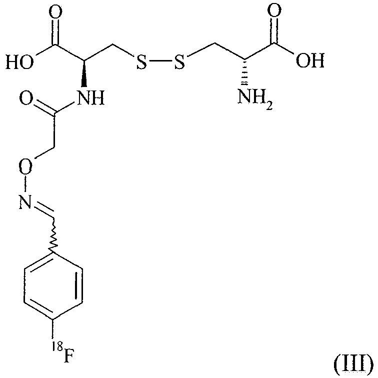

This compound is also referred to herein as monoAO-[¹⁸F]-FBA-Cystine.

Other examples of ¹⁸F-labeled cystine compounds include, to: [¹⁸F]fluoroethyl-cystine or [¹⁸F]FE-cystine having the structure V: 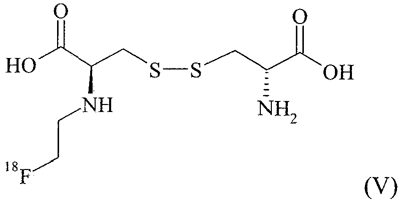 and [¹⁸F]fluoropropanamido-cystine or [¹⁸F]FP-cystine having the Structure VI:

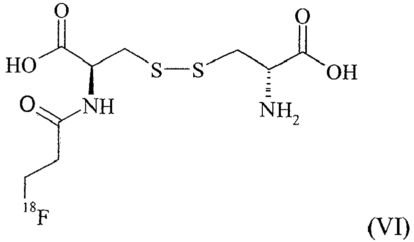

Imaging agents of the invention wherein the labeled cysteine compound comprises a radioisotopic label are suitably prepared from precursor compounds. A "precursor compound" comprises a derivative of the labeled cystine compound, designed so that chemical reaction with a convenient chemical form of the radioisotopic label occurs site-specifically; can be conducted in the minimum number of steps (ideally a single step); and without the need for significant purification (ideally no further purification), to give the desired labeled cysteine compound. Such precursors are synthetic and can conveniently be obtained in good chemical purity. The precursor compound may optionally comprise one or more protecting groups for certain functional groups. Protecting groups are well-known to those skilled in the art and are described in detail in 'Protective Groups in Organic Synthesis', Theorodora W. Greene and Peter G. M. Wuts, (Fouth Edition, John Wiley & Sons, 2007). The precursor compound is ideally provided in sterile, apyrogenic form. The precursor compound can accordingly be used for the preparation of a pharmaceutical composition comprising the imaging agent together with a biocompatible carrier suitable for mammalian administration. The precursor compound is also suitable for inclusion as a component in a kit for the preparation of such a pharmaceutical composition. In a preferred embodiment, the precursor compound is provided in solution and as part of a kit or of a cassette designed for use in an automated synthesis apparatus.

A particular precursor compound of the disclosure has structure X: 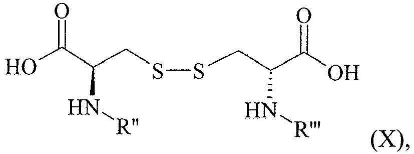 wherein one of R" and R'" is a precursor group and the other of R" and R'" is hydrogen, and wherein said precursor compound optionally comprises protecting groups on one or more of the hydroxy, carbonyl and amine functional groups. A "precursor group" is a chemical group which reacts with a convenient chemical form of the radioisotopic label to incorporate the radioisotopic label site-specifically Suitable such precursor groups are now described in the context of particular radioisotopic labels.

When the radioisotopic label of the imaging agent is ¹⁸F, the radiofluorine atom may form part of a fluoroalkyl or fluoroalkoxy group, since alkyl fluorides are resistant to in vivo metabolism. Alternatively, the radiofluorine atom may be attached via a direct covalent bond to an aromatic ring. Radiofluorination may be carried out via direct labelling using the reaction of ¹⁸F-fluoride with a precursor group that comprises a good leaving group, such as bromide, mesylate or tosylate. ¹⁸F can also be introduced by O-alkylation of a hydroxyl precursor group with [¹⁸F]-fluoroalkyl bromide, [¹⁸F]-fluoroalkyl mesylate or [¹⁸F]-fluoroalkyl tosylate. Nucleophilic displacement from an aryl diazonium salt, aryl nitro compound or an aryl quaternary ammonium salt are suitable routes to aryl-¹⁸F derivatives. A thorough review of ¹⁸F-labelling techniques may be found in "Chemistry of Fluorine-18 Radiopharmaceuticals" by Snyder and Kilbourn in Handbook of Radiopharmaceuticals, Ed. M.J. Welch and C.S. Redvanly (2003, John Wiley and Sons).

For radioiodination, the precursor compound preferably comprises a precursor group that is: an aryl iodide or bromide (to permit radioiodine exchange); an activated precursor compound aryl ring (e.g. a phenol group); an organometallic precursor compound (e.g. trialkyltin, trialkylsilyl or organoboron compound); or an organic precursor compound such as triazenes or a good leaving group for nucleophilic substitution such as an iodonium salt. Precursor compounds and methods of introducing radioiodine into organic molecules are described by Bolton (J. Lab. Comp. Radiopharm. 2002; 45: 485-528). Suitable boronate ester organoboron compounds and their preparation are described by Kabalka et al (Nucl. Med. Biol., 2002; 29: 841-843 and 2003; 30: 369-373). Suitable organotrifluoroborates and their preparation are described by Kabalka et al (Nucl. Med. Biol., 2004; 31: 935-938). Preferred precursor compounds for radioiodination comprise an organometallic precursor group, most preferably a trialkyltin.

Examples of aryl precursor groups to which radioactive iodine can be attached are given below:

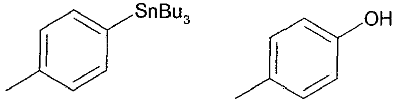

Both contain substituents which permit facile radioiodine substitution onto the aromatic ring. Alternative substituents containing radioactive iodine can be synthesised by direct iodination via radiohalogen exchange, e.g.

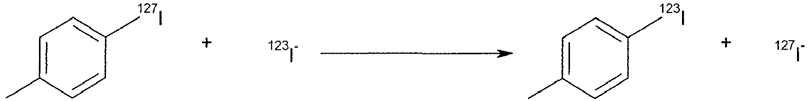

Radiobromination may be carried out using the precursor compounds described above for radioiodinated compounds. Kabalka and Varma have reviewed various methods for the synthesis of radiohalogenated compounds, including radiobrominated compounds (Tetrahedron 1989; 45(21): 6601-21).

A ¹¹C-labeled imaging agent of the invention may be synthesised in a straightforward manner by reacting a precursor compound which is a desmethylated version of the imaging agent with ¹¹C methyl iodide. It is also possible to incorporate ¹¹C by reacting a Grignard reagent of the particular hydrocarbon of the desired imaging agent with [¹¹C]CO₂ to obtain a ¹¹C reagent that reacts with an amine precursor group in the precursor compound to result in the ¹¹C-labelled imaging agent of interest. A Grignard reagent comprises a magnesium halide precursor group at the desired site of radiolabelling. As the half-life of ¹¹C is only 20.4 minutes, it is important that the ¹¹C labelling intermediate has high specific activity and, consequently, that it is produced using a reaction process which is as rapid as possible. A thorough review of such ¹¹C-labelling techniques may be found in Antoni et al "Aspects on the Synthesis of 11C-Labelled Compounds" in Handbook of Radiopharmaceuticals, Ed. M.J. Welch and C.S. Redvanly (2003, John Wiley and Sons).

The disulfide bond of structure I is reduced inside the cell and will no longer be a substrate for the xc- transporter so that the agent cannot leave the cell via this route. A fluorescent or radioisotope label (R) will be conjugated to an amine so that the resulting reduced intracellular agent will not be metabolized. The resulting agent therefore enters and is retained in the cells with an activated xc- transporter. A single-labeled cysteine compound has an H at the R' position, but an additional label may also be appended to the second amine at the R' position.

Using labeled cystine as an example, cystine is labeled at one of the free amines. When imported into the cell via the transporter, it will be reduced to labeled cysteine, which is no longer a substrate of the transporter and therefore could not be expelled from the cell by the same mechanism of entry. Because the amine is conjugated to the label, the labeled cysteine will not be a substrate for glutathione synthesis or protein synthesis and the label will be trapped in the cells.

In a wide variety of human tissues and cells examined, the xc- transporter is predominantly expressed brain, but also in pancreas and in cultured cell lines. The xc- transporter expression is very low in most tissues, but can be upregulated under conditions of oxidative stress and when cells are grown in culture. The xc-transporter is induced under a number of conditions, including apoptotic stimuli, oxidative stress, inflammation, cystine deprivation and chemotherapy resistance.

The imaging agent of the invention as suitably and preferably defined above is preferably provided as a pharmaceutical composition comprising the imaging agent together with a biocompatible carrier in a form suitable for mammalian administration. The pharmaceutical composition forms a separate aspect of the invention.

The "biocompatible carrier" is a fluid, especially a liquid, in which the imaging agent is suspended or dissolved, such that the pharmaceutical composition is physiologically tolerable, i.e. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is either isotonic or not hypotonic); an aqueous solution of one or more tonicity-adjusting substances (e.g. salts of plasma cations with biocompatible counterions), sugars (e.g. glucose or sucrose), sugar alcohols (e.g. sorbitol or mannitol), glycols (e.g. glycerol), or other non-ionic polyol materials (e.g. polyethyleneglycols, propylene glycols). The biocompatible carrier may also comprise biocompatible organic solvents such as ethanol. Such organic solvents are useful to solubilise more lipophilic compounds or formulations. Preferably the biocompatible carrier is pyrogen-free water for injection, isotonic saline or an aqueous ethanol solution. The pH of the biocompatible carrier for intravenous injection is suitably in the range 4.0 to 10.5.

These agents that are taken up into cells via the cystine/glutamate antiporter (xc- transporter system) may be used to image cellular oxidative stress in vivo, including without limitation, the imaging of pathologies or conditions that include cellular oxidative stress. Imaging applications that would benefit from these agents include, but are not limited to, chemotherapy treatment monitoring, ischemia/stroke, inflammation, traumatic brain injury and organ transplant monitoring.

Accordingly, in another aspect, the present invention relates to a method for imaging a biological sample having a cystine/glutamate transporter, said method comprising:
introducing into said biological sample via the cystine/glutamate transporter an imaging agent as defined in Claim 1; and
detecting said imaging agent using fluorescence microscopy, laser-confocal microscopy, cross-polarization microscopy, optical imaging, nuclear scintigraphy, positron emission tomography, or single photon emission computed tomography.

The biological sample is preferably an in vitro cell culture. In this case, the step of introducing the imaging agent of the invention is carried out by adding the imaging agent suspended in a suitable buffer to the in vitro cell culture, followed by incubation for a defined period of time, preferably at physiological temperature. The detecting step is subsequently carried out using fluorescence microscopy, laser-confocal microscopy, cross-polarization microscopy techniques well known to the skilled person. See for example "Principles of Fluorescence Microscopy" Third Edition 2006, Lakowitz, Ed.

Alternatively preferably, the biological sample is an intact mammalian subject. Where the biological sample is an intact mammalian subject, the imaging agent is preferably administered as the pharmaceutical composition of the invention. Preferably administration to said subject is carried out parenterally, and most preferably intravenously. The intravenous route represents the most efficient way to deliver the imaging agent throughout the body of the subject. Intravenous administration does not represent a substantial physical intervention or a substantial health risk. The imaging agent of the invention is preferably administered as the pharmaceutical composition of the invention, as defined herein.

Where the biological sample is an intact mammalian subject, the detecting step of the method of imaging is preferably carried out using positron emission tomography or single photon emission tomography, preferably wherein said imaging agent comprises a cysteine compound of structure II or III as defined above. For positron emission tomography it is preferred that said imaging agent comprises a labeled cystine compound of structure III as defined above.

In a further aspect, the present invention provides a method for detecting oxidative stress in cells comprising:
introducing an imaging agent as defined in Claim 1 into a cystine/glutamate antiporter of the cells;
allowing the intracellular labeled cysteine compound to be reduced into a labeled cysteine; and,
detecting the labeled cysteine in the cell.

Preferably for said method of imaging and for said method for detecting oxidative stress, said detecting is carried out in apoptotic cells.

Following are examples used to illustrate various embodiments of the imaging agents and methods of use.

### Example 1

Human Jurkat cells were cultured with or without 1uM staurosporine for 16 hours in an in vitro assay for apoptotic cells and stained with propidium iodide(PI) and Cy5-AnnexinV (Annexin). Typically ∼30-50% of cells were found to be in some stage of apoptosis or necrosis. Flow cytometry was used to identify population of cells defined as normal (PI and Annexin negative), early apoptotic (PI negative, Annexin positive), and late apoptosis and necrosis (PI and Annexin positive). The results are shown in FIGs. 2A and 2B. FIG 2A is a forward scatter/side scatter plot showing some late apoptotic cells (light gray) with a different granularity than non-apoptotic cells (darker gray). In FIG 2B, AnnexinV and PI negative cells are shown in quadrant D3 and represent non-apoptotic cells. Cells in quadrant D4 indicate early apoptotic cells that are positive for AnnexinV staining, but negative for PI staining. Cells in quadrant D2 indicate late apoptotic and necrotic cells that are positive with both AnnexinV and PI staining.

### Example 2 (comparative)

Jurkat cells were incubated with (FIG 3B) and without (FIG 3A) 1 µM staurosprine (STN) for 16-18 hours, stained with Annexin V-Cy5 and propidium iodide, and incubated with DBC for 30 minutes. DBC is a commercially available product from Invitrogen (catalog #B20340), which is sold for the purpose of reversible thiol labeling of nucleotides, proteins and cells via a disulfide exchange reaction at acidic conditions. DBC has the structure shown below.

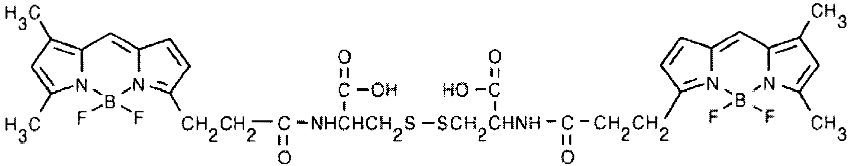

In FIG 3A, untreated cells show some low intensity fluorescence corresponding to uptake or nonspecific binding of the DBC molecule. In FIG 3B, staurosporine induced cells have a subpopulation of cells with high intensity DBC staining and this correlated primarily to the late apoptotic/necrotic cells (+AnnexinV, + PI). A population of cells with intermediate DBC staining also appeared, which correlates well with the early apoptotic cells (+AnnexinV, -PI). To more specifically address the role of the the xc- transporter in this apoptotic cell labeling assay, Jurkat cells were also incubated with sulfasalazine, a potent specific inhibitor of the xc-transporter. Cells were treated as in FIG 2B except that a specific inhibitor of the cystine/glutamate transporter (sulfasalazine, sasz) was added at the same time as the DBC (FIG 3C). In FIG 3C, a population of cells with intermediate DBC staining was found, which correlated well only with late apoptotic cells (+AnnexinV, + PI). In FIG 3D, cells were categorized as normal, early apoptotic, or late apoptotic as in Example 1, and the percentage of cells with increased DBC staining was recorded for each category. Early and late apoptotic cells were labeled with DBC, but this was only inhibited by sasz in the early apoptotic cells. As such DBC does label early apoptotic cells via activity of the cystine-glutamate transporter and acts as a transporter substrate even though it is conjugated to two fluorophores via the amines of cystine. This data shows that the uptake seen in early apoptotic cells with DBC is dependent upon the xc- transporter, rather than some other mechanism.

### Example 3 (comparative)

The apoptotic Jurkat cell uptake of DBC shows not only that the xc-transporter is activated in cells undergoing oxidative stress (and in this case apoptosis), but also that the xc- transporter is promiscuous enough to allow this substrate (cystine) with amine appended green fluorophores (bodipyFL) into the cell. A labeled cystine compound was synthesized with a red fluorophore (bodipy650) to further determine the promiscuity of the transporter. In a side-by-side comparison to the bodipyFL labeled cystine, DBC-650 did not appear to label apoptotic cells any more than normal cells, as shown in FIGs. 4A and 4B, indicating that some labels are not sufficient for maintaining xc- transporter substrate status. In this example, the bodipy-650 fluorophore and associated linker are larger than that of DBC (FIG 4A), and therefore size represents one limitation of what groups are appended to the amines while retaining the ability to pass through the transporter.

### Example 4

From the previous example, it is important to keep the size of the label small. Smaller labeled cystine compounds serve as more appropriate substrates than larger labeled cystine compounds such as DBC-650 and DBC. Therefore, a smaller fluorescent cystine compound was synthesized, with the fluorescent label Bodipy at only one of the amines, MonoBodipyCystine (MBC). This molecule was created using commercially available cystine and BODIPY-FL succinimidyl ester (Invitrogen, D2184), purified by reverse phase- HPLC and analyzed by mass spectrometry to confirm the correct product. MBC has the structure shown below.

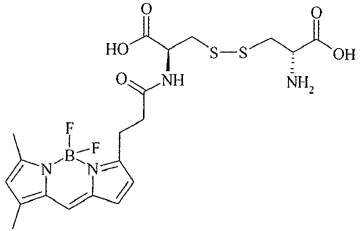

Jurkat cells were incubated with 1 µM STN for 16-18 hours, stained with Annexin V-Cy5 and propidium iodide, and incubated with MBC for 30 minutes, without (FIG 5A) and with (FIG 5B) the addition of the cystine/glutamate inhibitor (sasz). In FIG 5A, MBC labeling is visualized as a subpopulation of cells with a high intensity fluorescence shift, which corresponds well with both early and late apoptotic cells (FIG 5C). No intermediate population was evident, suggesting that MBC labeled early apoptotic cells with greater intensity than DBC. In FIG 5B, the addition of sulfasalazine results in fewer labeled cells and with less of a shift in fluorescence intensity. This population correlated well with late apoptotic cells and did not correlate well with early apoptotic cells (FIG 5C). In this example, MBC is smaller than DBC (with only one label) and more closely resembles the natural transporter substrate cystine; resulting in an agent with improved performance for labeling early apoptotic cells. With MBC, there is less background uptake in normal cells, without sacrificing the magnitude of increase in fluorescence intensity for apoptotic cells, even though there is one less fluorophore per cystine compound.

### Example 5

Agents that contain a reducible disulfide bond should be reduced inside of the intracellular compartment and therefore would not be a substrate for efflux by the cystine/glutamate transporter and would result in trapping the label inside the cells. To show this action, a DiBodipy Cystathionine (DBCystathionine) agent was formulated with the following structure.

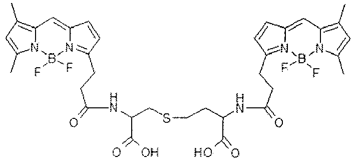

Also used as a negative control, was Bodipy-FL C5, which is available commercially from Invitrogen that has the following structure.

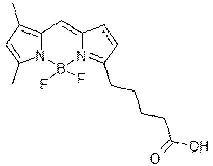

FIG 6A shows a comparison of Bodipy staining of normal, early apoptotic and late apoptotic Jurkat cells with DBC, DBC(650), DBCystathionine, MBC and the negative control (Bodipy-FL C5). DBC, DBCystathionine, and MBC stain early apoptotic cells, DBC(650) only stains late apoptotic cells, and the negative control does not show any shift in fluorescence for apoptotic cells. From this data, DBC, DBCystathionine, and MBC are all equivalent labels for apoptotic cells. However, in FIG 6B, the fold shift in fluorescence intensity of the early apoptotic cells is compared to normal cells for each agent. It is clear that MBC provides more intense staining than the other fluorescent agents used.

### Example 6

As evident from the previous examples, it is beneficial to have a single small label. Smaller labeled cystine compounds serve as more appropriate substrates than larger labeled cystine compounds such as DBC-650. Therefore, labeled cystine compounds were synthesized with a single amine conjugated to [¹⁸F]aminoxy-fluorobenzadehyde (monoAO-[¹⁸F]-FBA-Cystine), and may be used for PET imaging applications. First the aminoxy-cystine precursor (monoAO-cystine) was synthesized, with the following structure.

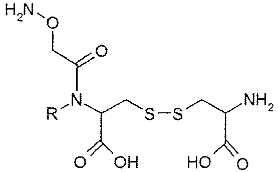

Generally, the monoAO-cystine was then conjugated to [¹⁸F]Fluorobenzaldehyde to yield monoAO-[¹⁸F]-FBA-Cystine with the following structure.

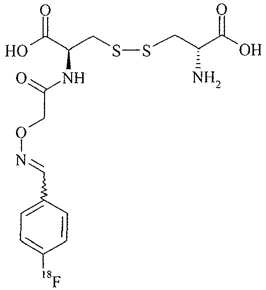

A more specific, non-limiting example of the method for synthesizing the monoAO-[¹⁸F]-FBA-Cystine is provided as follows.

All reactions were performed either under a nitrogen atmosphere or in a crimp-top sealed vial purged with nitrogen. Kryptofix 222 (Aldrich) and K2CO3 (EMD Science) were purchased and used as received. Optima™-grade acetonitrile was used as both HPLC and reaction solvents.

[¹⁸F]KF (40mCi.mL-1 (1480 MBq.mL-1) in purified water) was obtained from either IBA Molecular (Albany, NY) or PETNET Solutions (Albany, NY) and used as received. The [¹⁸F]fluoride was first immobilized on a Chromafix 30-PS-HCO3 anion exchange cartridge (ABX, Radeberg, Germany), then eluted into a drydown vessel with a 1 mL, 4:1 mixture of acetonitrile:distilled deionized water (ddH₂O) containing Kryptofix K222 (376 g.mol-1, 8 mg, 2.13x10⁻⁵ mol) and potassium carbonate (138.2 g.mol-1, 2.1 mg, 1.52x10⁻⁵ mol). The solvent was removed under partial vacuum and a flow of nitrogen with gentle heating (∼ 45 °C) (∼15 min). The source vial and anion exchange cartridge were then washed with 0.5mL of acetonitrile containing K222 (8 mg) and the reaction mixture again brought to dryness under partial vacuum and gentle heating (∼10 min). The reaction vessel was repressurized with nitrogen and the azeotropic drydown repeated twice with an additional 0.5mL of acetonitrile. 4-formyl-N,N,N-trimethylanilinium triflate (313.30 g.mol-1, 3.1 mg, 9.89x10⁻⁶ mol) was dissolved in 0.35 mL of anhydrous DMSO (Acros) and added directly to the reaction vessel containing the [¹⁸F]KF.K222, K2CO3. The reaction mixture was heated to 90°C for 15 min and immediately cooled and quenched with 3 mL of distilled, deionized H₂O (ddH₂O). This mixture was subsequently passed through a cation exchange cartridge (Waters SepPak Light Accell Plus CM), diluted to 10 mL with ddH2O, and loaded onto a reverse phase C18 SepPak (Waters SepPak Plus C18). The SepPak was flushed with 10 mL of ddH₂O then purged with 30 mL of air. [¹⁸F]4-fluorobenzaldehyde ([¹⁸F]FBA), was eluted in 1.0 mL of methanol.

Separately, a high recovery vial (2mL, National Scientific) was charged with mono-aminoxy cystine (386.27g.mol⁻¹, 2.7mg, 6.99x10⁻⁶ mol). The solid was suspended in 250 µL of ddH₂O and 8 µL of trifluoroacetic acid. 500 µL of [¹⁸F]FBA in methanol (see above) was transferred to the reaction vial. The vessel was capped, crimped, placed in a heating block (activity at start of reaction 4.66 mCi/172 MBq) and maintained at 60°C for 15 minutes; at which point a small aliquot (<5 µL) was removed for analytical HPLC analysis. 250 µL of ddH₂O with 0.1% TFA was used to dilute the solution to approx. 1000 µL, giving a final composition of 1:1 ddH₂O:MeOH in preparation for semi-preparative HPLC purification. [¹⁸F]FB-Cystine was isolated and purified by semi-preparative HPLC. The HPLC fraction containing the product (0.409 mCi/15.1MBq) was diluted 5:1 with ddH₂O and subsequently immobilized on a tC18 Plus Sep Pak (Waters). The SepPak was flushed first with 5 mL of ddH₂O then 30 mL of air. [¹⁸F]FB-Cys (0.17mCi, 6.3 MBq) was isolated in a minimal amount of DMSO by first eluting the void volume (approx. 0.5mL) followed by collecting 250 to 300 µL of eluent in a separate flask. RP-HPLC analysis was performed on the isolated product in order to establish radiochemical and chemical purity. Typically, 10 µL of a 0.1µCi/µL solution was injected for post formulation analysis. Isolated radiochemical yield was 3.6% (6.6% decay corrected from addition of [¹⁸F]FBA) and radiochemical purity of 96.8%.

Analytical HPLC conditions: Analysis performed on an HP Agilent 1100 with a G1311A QuatPump, G1313A autoinjector with 100µL syringe and 2.0mL seat capillary, Phenomenex Gemini C18 column (4.6mm×150mm), 5µ, 100Å (S/N 420477-10), G1316A column heater, G1315A DAD and Ramon Star ··· GABI gamma-detector. 95:5 ddH₂O:CH₃CN with 0.05% TFA, Solvent B: CH₃CN with 0.05% TFA. Gradient elution: 0 min. 0%B, 1 min. 15%B, 10min. 31%B, 10.5 min. 100%B, 13.5 min. 100%B, 14 min. 0%B, 17 min. 0%B. (TR ∼ 7.1 min)

Semipreparative HPLC conditions: Purification was performed on a Jasco LC with a DG-2080-54 4-line Degasser, an MX-2080-32 Dynamic Mixer and two PU-2086 Plus Prep pumps, an AS-2055 Plus Intelligent autoinjector with large volume injection kit installed, a Phenomenex 5µ. Luna C18(2) 100Å, 250 x. 10 mm, 5 µ . column with guard (S/N 295860-1, P/N 00G-4252-N0), an MD-2055 PDA and a Carroll & Ramsey Associates Model 105S Analogue Ratemeter attached to a solid-state SiPIN photodiode gamma detector. Gradient elution: 0 min. 0%B, 3 min. 20%B, 42 min. 70%B, 42.5 min. 100%B, 46 min. 100%B, 50 min. 0%B, Solvent A: ddH₂O:CH₃CN with 0.05% TFA, Solvent B: CH3CN with 0.05% TFA. (TR ∼ 14.7 min)

The monoAO-[¹⁸F]-FBA-Cystine was used in an in vitro cell uptake assay, wherein monoAO-[¹⁸F]-FBA-Cystine was incubated with cultured cells for 30 minutes and washed twice with saline before lysing the cells with IN NaOH and collection for analysis in a gamma counter. FIG 7, shows the uptake of monoAO-[¹⁸F]-FBA-Cystine in two different cell lines, indicating differential basal expression and activity of the cystine/glutamate transporter in Jurkat and A549 cell lines.

A pharmacokinetic profile of this molecule, which was formulated into 7% ethanol in saline, was established in normal mice and is shown in FIGs. 8A and 8B. The naive Balb/c mice were injected ∼15 uCi with ¹⁸F-cystine and time points were taken at 5, 30, 120 min post injection. FIG 8A shows the biodistribution results in naïve Balb-c mice, % of injected dose (%ID). Clearance from the body is largely due to renal excretion as shown by the profile of the kidney, bladder and urine. FIG 8B shows the %ID/gram of the same data shown in FIG 8A. In two mice, apoptosis was induced with an injection of anti-Fas antibody two hours before injection of the radiotracer. Two animals were investigated that received anti-Fas antibody injections 2 hours prior to the monoAO-[¹⁸F]-FBA-Cystine injection to induce apoptosis in the liver. While the number of animals investigated thus far is low, both show liver uptake above the control animals.

FIG 8C shows a similar biodistribution results in %ID/gram from a study done in nude mice with A549 tumor xenografts. At 120 minute and 240 minute time points the radiotracer has cleared sufficiently to detect more %ID/gram in the tumor than in blood or other tissues (except kidney and bladder). Tumor to blood ratios are shown for each time point in FIG 8D. These results suggest that radiolabeled cystine compounds can be used for the detection of cystine/glutamate transporter activity *in vivo.*

FIG 9 shows the stability of the monoAO-[¹⁸F]-FBA-Cystine molecule over time in saline. As shown, there was not any change in the gamma-trace profile over a 4-hour period.

FIG 10 shows the monoAO-[¹⁸F]-FBA-Cystine in a PET image in a naive mouse at 60 minutes post injection, showing clearance primarily through the kidneys and bladder.

As another example, the cystine may be labeled with ¹²³I-iodobenzaldehyde, which similar fluorobenzaldehyde, may be prepared by first adding [¹²³I]4-iodobenzaldehyde ([¹²³I]IBA) to a high recovery vial (2 mL, National Scientific) containing the AO-Cys , 2.5 mg). The reaction commences by dissolving the polypeptide in 0.5 mL of ddH₂O and adding 8 µL of trifluoroacetic acid followed by the addition of [¹²³I]IBA in 0.5 mL of methanol. The vessel is capped, crimped, placed in a heating block and maintained at 60°C for 15 minutes; removing a small aliquot (<5 µL) for analytical HPLC analysis is done to assess the status of the reaction. [¹²³I]IB-Cystine is isolated and purified by semi-preparative HPLC. The HPLC fraction containing the product is further diluted (5:1) with ddH₂O and the product subsequently immobilized on a tC18 Plus Sep Pak (Waters). Flushing the SepPak first with 5 mL of ddH₂O then 30 mL of air gives the [¹²³I]IB-Cystine in a minimal amount of ethanol by first eluting the void volume (approx. 0.5mL) followed by collecting 250 to 300 µL of eluent in a separate flask. RP-HPLC analysis is then performed on the isolated product to establish radiochemical and chemical purity.

## Claims

1. An imaging agent comprising a labeled cystine compound having structure I: 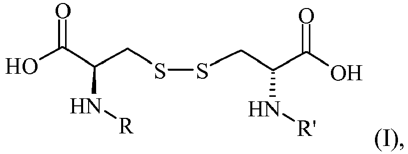 wherein one of R and R' comprises a label selected from a Bodipy fluorescent dye or a radioisotopic label, and the other of R and R' is hydrogen.

2. The imaging agent as defined in Claim 1, wherein the labeled cystine compound has structure II: 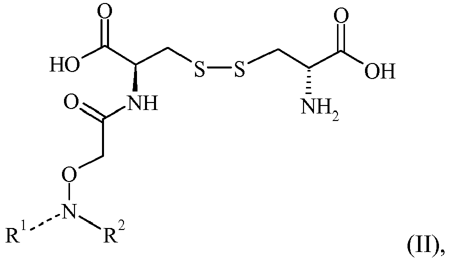 wherein:
the dotted bond represents either a single bond or a double bond;
R¹ comprises a radioisotopic label;
R² is hydrogen when the dotted bond is a single bond; and,
R² is absent when the dotted bond is a double bond.

3. The imaging agent as defined in Claim 1 or Claim 2 wherein said radioisotopic label is selected from ¹¹C, ¹³N, ¹⁵O, ¹⁷F, ¹⁸F, ⁷⁵Br, ⁷⁶Br, ¹²⁴I, ¹²³I, ¹³¹I and ⁷⁷Br.

4. The imaging agent as defined in Claim 3, wherein said radioisotopic label is ¹⁸F and wherein said labeled cystine compound is selected from the compounds of structures III, V or VI: 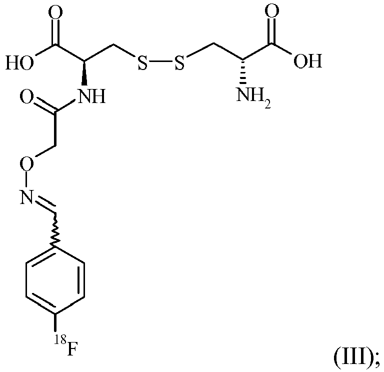 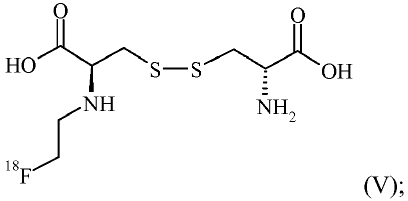 and,

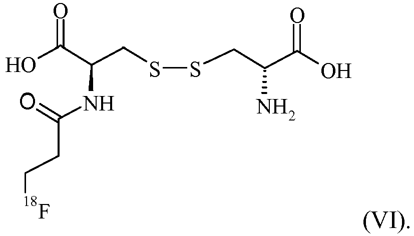

5. The imaging agent as defined in Claim 1, wherein the labeled cystine compound comprises a fluorescent label and has structure IV:

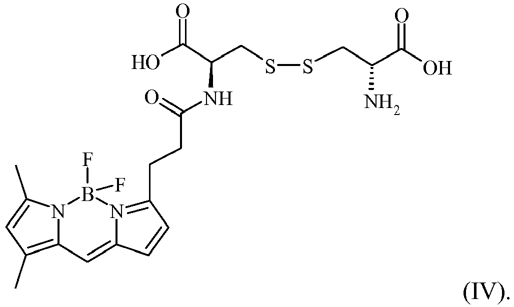

6. A method for imaging a biological sample having a cystine/glutamate transporter, said method comprising:
introducing into said biological sample via the cystine/glutamate transporter an imaging agent as defined in Claim 1; and
detecting said imaging agent using fluorescence microscopy, laser-confocal microscopy, cross-polarization microscopy, optical imaging, nuclear scintigraphy, positron emission tomography, or single photon emission computed tomography.

7. The method as defined in Claim 6 wherein said biological sample is an in vitro cell culture.

8. The method as defined in Claim 7, wherein said detecting step is carried out using fluorescence microscopy, laser-confocal microscopy, or cross-polarization microscopy.

9. The method as defined in Claim 6 wherein said biological sample is an intact mammalian subject.

10. The method as defined in Claim 9 wherein said imaging agent is as defined in Claim 2 or Claim 3 and wherein said detecting step is carried out using positron emission tomography or single photon emission tomography.

11. The method as defined in Claim 10 wherein said imaging agent comprises a labeled cystine compound as defined in Claim 4 and wherein said detecting step is carried out using positrion emission tomography.

12. A method for detecting oxidative stress in cells comprising:
introducing an imaging agent as defined in any one of Claims 1-5 into a cystine/glutamate antiporter of the cells;
allowing the intracellular labeled cystine compound to be reduced into a labeled cysteine; and,
detecting the labeled cysteine in the cell.

13. The method as defined in Claim 12 wherein said cells are apoptotic cells.

14. A pharmaceutical composition comprising the imaging agent as defined in any one of Claims 1-5 together with a biocompatible carrier in a form suitable for mammalian administration.

## Patentansprüche

1. Bildgebungsmittel, umfassend eine markierte Cystinverbindung mit der Struktur I: 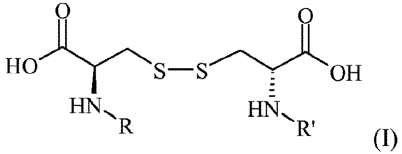 wobei einer von R und R' eine Markierung umfasst, die ausgewählt ist aus einem fluoreszierenden Bodipy-Farbstoff oder einer Radioisotopen-Markierung, und der andere von R und R' Wasserstoff ist.

2. Bildgebungsmittel nach Anspruch 1, wobei die markierte Cystinverbindung die Struktur II aufweist: 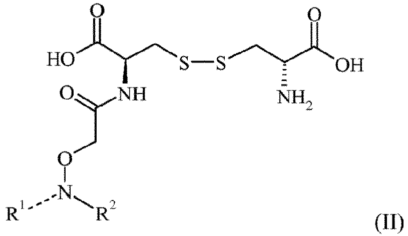 worin:
die gestrichelte Bindung entweder eine Einfachbindung oder eine Doppelbindung darstellt;
R¹ eine Radioisotopen-Markierung umfasst;
R² Wasserstoff ist, wenn die gestrichelte Bindung eine Einfachbindung ist; und
R² fehlt, wenn die gestrichelte Bindung eine Doppelbindung ist.

3. Bildgebungsmittel nach Anspruch 1 oder Anspruch 2, wobei die Radioisotopen-Markierung ausgewählt ist aus ¹¹C, ¹³N, ¹⁵O, ¹⁷F, ¹⁸F, ⁷⁵Br, ⁷⁶Br, ¹²⁴I, ¹²³I, ¹³¹I und ⁷⁷Br.

4. Bildgebungsmittel nach Anspruch 3, wobei die Radioisotopen-Markierung ¹⁸F ist und wobei die markierte Cystinverbindung ausgewählt ist aus den Verbindungen der Strukturen III, V oder VI: 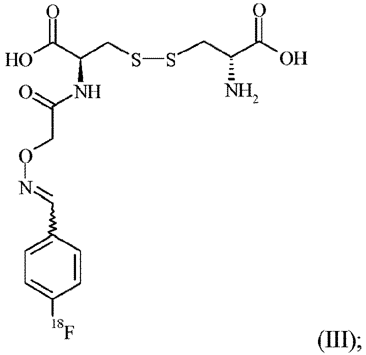 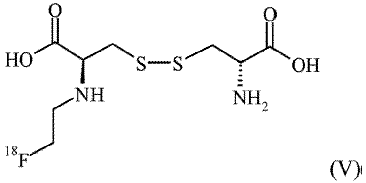 und,

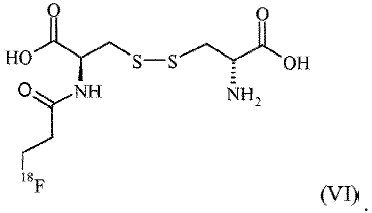

5. Bildgebungsmittel nach Anspruch 1, wobei die markierte Cystinverbindung eine fluoreszierende Markierung umfasst und die Struktur IV aufweist:

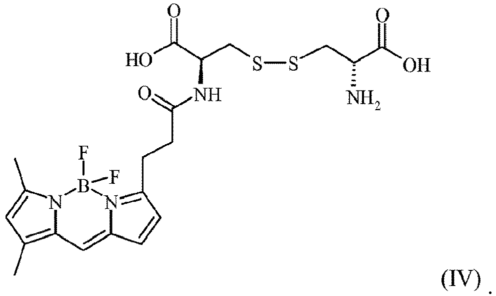

6. Verfahren zur Bildgebung einer biologischen Probe mit einem Cystin/Glutamat-Transporter, wobei das Verfahren umfasst:
Einbringen eines Bildgebungsmittels nach Anspruch 1 in die biologische Probe über den Cystin/Glutamat-Transporter; und
Erfassen des Bildgebungsmittels unter Verwendung von Fluoreszenzmikroskopie, Laserkonfokalmikroskopie, Kreuzpolarisationsmikroskopie, optischer Bildgebung, Kernszintigraphie, Positronen-Emissions-Tomographie oder Einzelphotonen-Emissions-Computertomographie.

7. Verfahren nach Anspruch 6, wobei die biologische Probe eine in vitro-Zellkultur ist.

8. Verfahren nach Anspruch 7, wobei der Erfassungsschritt unter Verwendung von Fluoreszenzmikroskopie, Laserkonfokalmikroskopie oder Kreuzpolarisationsmikroskopie durchgeführt wird.

9. Verfahren nach Anspruch 6, wobei die biologische Probe ein intakter Säuger ist.

10. Verfahren nach Anspruch 9, wobei das Bildgebungsmittel nach Anspruch 2 oder 3 definiert ist und wobei der Erfassungsschritt unter Verwendung der Positronen-Emissions-Tomographie oder der Einzelphotonen-Emissions-Tomographie durchgeführt wird.

11. Verfahren nach Anspruch 10, wobei das Bildgebungsmittel eine markierte Cystinverbindung nach Anspruch 4 umfasst und wobei der Erfassungsschritt unter Verwendung der Positronen-Emissions-Tomographie durchgeführt wird.

12. Verfahren zum Erfassen von oxidativem Stress in Zellen, umfassend:
Einbringen eines Bildgebungsmittels nach einem der Ansprüche 1-5 in einen Cystin/Glutamat-Antiporter der Zellen;
Erlauben, dass die intrazellulär markierte Cystinverbindung zu einem markierten Cystein reduziert wird; und,
Erfassen des markierten Cysteins in der Zelle.

13. Verfahren nach Anspruch 12, wobei die Zellen apoptotische Zellen sind.

14. Pharmazeutische Zusammensetzung, umfassend das Bildgebungsmittel nach einem der Ansprüche 1-5 zusammen mit einem biokompatiblen Träger in einer für die Verabreichung an Säuger geeigneten Form.

## Revendications

1. Agent d'imagerie comprenant un composé de cystine marqué ayant la structure I : 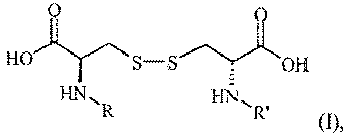 dans laquelle l'un parmi R et R' comprend un marqueur choisi parmi un colorant fluorescent Bodipy ou un marqueur radio-isotopique, et l'autre parmi R et R' est l'hydrogène.

2. Agent d'imagerie selon la revendication 1, dans lequel le composé de cystine marqué a la structure II : 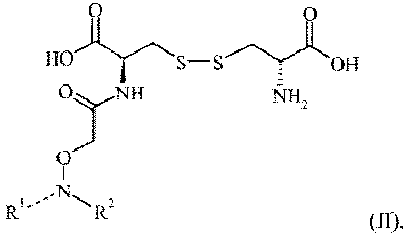 où :
la liaison en pointillés représente une liaison simple ou une liaison double ;
R¹ comprend un marqueur radio-isotopique ;
R² est l'hydrogène lorsque la liaison en pointillés est une liaison simple ; et
R² est absent lorsque la liaison en pointillés est une liaison double.

3. Agent d'imagerie selon la revendication 1 ou la revendication 2, dans lequel ledit marqueur radio-isotopique est choisi parmi ¹¹C, ¹³N, ¹⁵O, ¹⁷F, ¹⁸F, ⁷⁵Br, ⁷⁶Br, ¹²⁴I, ¹²³I, ¹³¹I et ⁷⁷Br.

4. Agent d'imagerie selon la revendication 3, dans lequel ledit marqueur radio-isotopique est ¹⁸F et dans lequel ledit composé de cystine marqué est choisi parmi les composés de structures III, V ou VI : 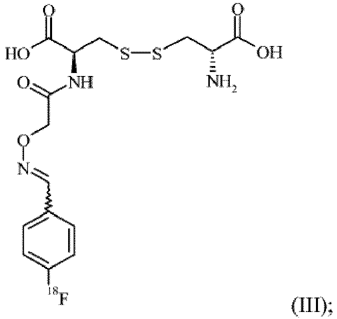 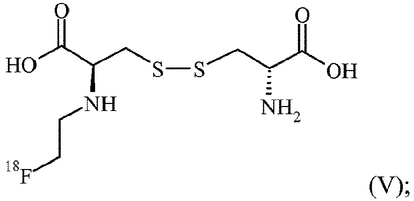 et,

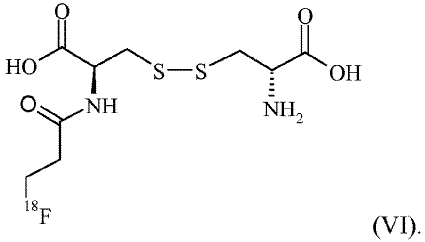

5. Agent d'imagerie selon la revendication 1, dans lequel le composé de cystine marqué comprend un marqueur fluorescent et à la structure IV :

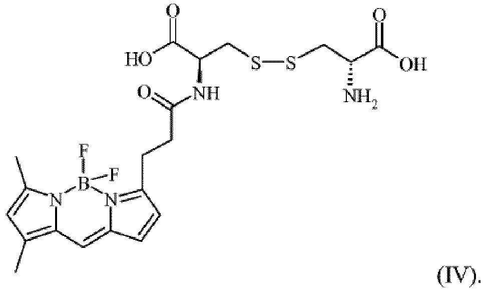

6. Procédé d'imagerie d'un échantillon biologique ayant un transporteur de cystine/glutamate, ledit procédé comprenant :
introduire dans ledit échantillon biologique par l'intermédiaire du transporteur de cystine/glutamate un agent d'imagerie tel que défini dans la revendication 1 ; et
détecter ledit agent d'imagerie en utilisant la microscopie à fluorescence, la microscopie confocale à laser, la microscopie à polarisation croisée, l'imagerie optique, la scintigraphie nucléaire, la tomographie à émission de positrons ou la tomographie calculée à émetteur de photon unique.

7. Procédé selon la revendication 6, dans lequel ledit échantillon biologique est une culture cellulaire in vitro.

8. Procédé selon la revendication 7, dans lequel ladite étape de détection est réalisée en utilisant une microscopie à fluorescence, une microscopie confocale à laser ou une microscopie à polarisation croisée.

9. Procédé selon la revendication 6, dans lequel ledit échantillon biologique est un sujet mammifère intact.

10. Procédé selon la revendication 9, dans lequel ledit agent d'imagerie est tel que défini dans la revendication 2 ou la revendication 3 et dans lequel ladite étape de détection est effectuée en utilisant une tomographie par émission de positrons ou une tomographie par émission de photons unique.

11. Procédé selon la revendication 10, dans lequel ledit agent d'imagerie comprend un composé de cystine marqué tel que défini dans la revendication 4, et dans lequel ladite étape de détection est effectuée en utilisant une tomographie à émission de positrons.

12. Procédé de détection de stress oxydant dans des cellules comprenant :
l'introduction d'un agent d'imagerie tel que défini dans l'une quelconque des revendications 1 à 5 dans un antiporteur cystine/glutamate des cellules ;
permettre au composé de cystine marqué intracellulaire d'être réduit en une cystéine marquée ; et
détecter la cystéine marquée dans la cellule.

13. Procédé selon la revendication 12, dans lequel lesdites cellules sont des cellules apoptotiques.

14. Composition pharmaceutique comprenant l'agent d'imagerie tel que défini dans l'une quelconque des revendications 1 à 5, conjointement avec un support biocompatible sous une forme appropriée pour une administration à un mammifère.
